# EUROPEAN PATENT APPLICATION

(11) **EP 4 209 265 A1**
(43) Date of publication of application: **12.07.2023**
(21) Application number: 22150553.0
(22) Date of filing: 07.01.2022
(51) Int. Cl.: B01J 13/18, A01N 25/28, A23K 40/30, A23P 10/30, A61K 8/11, A61K 9/48, C11D 3/50

(54) **BIODEGRADABLE CHITOSAN MICROCAPSULES**

(71) Applicant: International Flavors & Fragrances Inc., New York, NY 10019 (US)
(72) Inventor: LEI, Yabin, Union Beach, NJ, 07735 (US); GABBARD, Ronald, Union Beach, NJ, 07735 (US); HACKENBERG, Jason, Union Beach, NJ, 07735 (US); MERTZ, Gary, Union Beach, NJ, 07735 (US); POPPLEWELL, Lewis Michael, Union Beach, NJ, 07735 (US); SASAKI, Takashi, Union Beach, NJ, 07735 (US); TRAN, Tram, Union Beach, NJ, 07735 (US); YOUNG, Timothy, Union Beach, NJ, 07735 (US); ZHANG, Yi, Union Beach, NJ, 07735 (US)
(74) Representative: DuPont EMEA

(57) **Abstract**

Biodegradable core-shell microcapsules with a shell composed of a biopolymer, e.g., chitosan, which is cross-linked with an isocyanate and a tannic acid, in particular hydrolyzed tannic acid, are provided, as is a process for preparing the biodegradable core-shell microcapsules and consumer products containing the same.

## Description

### BACKGROUND OF THE INVENTION

Current commercial fragrance capsules including melamine-formaldehyde and polyurea are made from synthetic materials, which are not readily biodegradable. This is an issue for several reasons. Firstly, consumers are demanding more environmentally friendly products. Secondly, due to new regulations, for example from the European Chemicals Agency (ECHA), there will be a ban on the use of microplastics in a variety of consumer goods products (e.g., cosmetics, detergents, etc.). As a result, there is an ever-increasing demand for fragrance delivery technologies where the capsule wall material is more biodegradable and/or sustainable, and will satisfy the requirements set out in any currently existing and newly proposed regulations banning the use of microplastics.

Gelatin-based core-shell microcapsules have been described, which are indicated as being biodegradable. See WO 2021/122636 A1 (Firmenich) and WO 2021/122633 A1 (Firmenich). Further, microcapsules composed of natural polymers such as chitosan, carrageenan or gum arabic have been suggested. See WO 2021/018947A1 (Firmenich), WO 2021/122630 A1 (Firmenich), WO 2021/116306 A1 (Firmenich), and WO 2020/194910 A1 (Fujifilm). However, the degree or rate of biodegradability of these capsules is not described. Even assuming, arguendo, that the microcapsules disclosed herein above may pass any current regulations (e.g., the OECD301F or OECD310 tests), they may still not be 'truly' biodegradable and fully ECHA compliant. By that we mean that the microcapsules may not satisfy the requirements under the proposed ECHA regulations that materials forming the microcapsule walls are not to be considered a "blend" of biodegradable materials *(i.e.,* biopolymers) and non-biodegradable materials. Alternatively, if the microcapsule walls are considered a blend then all components of the blend would also meet the requirements for biodegradability (e.g., minimally at least the OECD301F or OECD310 tests).

Accordingly, microcapsules composed of sustainable, and/or biodegradable materials, which are chemically stable and/or deliver active materials such as fragrances in consumer products are still needed in the industry. Preferably, the materials used to form the microcapsule do not form a blend of biodegradable materials and non-biodegradable materials. Alternatively, if the materials used to form the microcapsule walls do form a blend, then not a significant amount (*i.e*., ≤ 10%, ≤ 5%, ≤ 3%, ≤ 1% or ≤ 0.5%) of non-biodegradable materials, or all components of the blend would also meet the requirements for biodegradability. The microcapsules preferably also provide acceptable fragrance profile and performance in use. The present invention satisfies this need and other needs in the industry.

### SUMMARY OF THE INVENTION

This invention is based on the discovery that biodegradable and/or sustainable microcapsule shells can be prepared by cross-linking biopolymers such as chitosan with a polyfunctional isocyanate and a tannic acid.

Accordingly, this invention provides biodegradable core-shell microcapsules, wherein: (i) the shell comprises chitosan (e.g., a fungal chitosan) cross-linked with a polyfunctional isocyanate having at least two isocyanate functional groups and a tannic acid, wherein the tannic acid is hydrolyzed tannic acid, unhydrolyzed tannic acid, or a combination thereof, preferably hydrolyzed tannic acid; and (ii) the core comprises an active material; wherein the shell of the microcapsules has a biodegradation rate of at least 20%, 30%, 40%, 50%, 60%, 65%, 70%, 75%, 80%, 85%, 90%, 95% or 98%, within 60 days according to OECD301F or OECD310, preferably a biodegradation rate of at least 60% within 60 days according to OECD301F or OECD 310. In some aspects, the shell further includes carrageenan, gum arabic (gum acacia), or a combination thereof. In other aspects, the polyfunctional isocyanate is a biuret, isocyanurate, allophanate, uretdione, oligomeric hexamethylene diisocyanate, or a combination thereof. Preferably, the isocyanate is dispersed in a solvent. In yet other aspects, the shell is further cross-linked with an amino acid, preferably the amino acid is selected from the group consisting of lysine, arginine, ornithine, cysteine, serine, threonine, asparagine, glutamine and a combination thereof, more preferably the amino acid is lysine.

The active material is a fragrance, flavor, agricultural active, pesticide, pharmaceutical active, nutraceutical active, animal nutrition active, food active, microbio active, malodor counteractant, and/or cosmetic active, preferably a fragrance. The invention is also based, *inter alia*, on the discovery of High Performance fragrance ingredients composed of certain Ultra High-Impact fragrance ingredients and High-Impact fragrance ingredients to deliver improved perceived intensity, perceived longevity and/or perceived fidelity of the fragrance profile at the various "touch points" (e.g., opening a fabric conditioner container, damp clothes upon opening a washing machine after washing laundry, opening a laundry dryer after drying laundry, drying clothes on drying frame and wearing laundered clothes) associated with the laundry experience. Preferably, the fragrance comprises at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2, and optionally at least one additional fragrance ingredient.

A process for making biodegradable core-shell microcapsules and biodegradable core-shell microcapsules obtainable by the process are also provided. In accordance with this method, an aqueous solution comprising chitosan is emulsified with an oil phase comprising an active material to form an emulsion. Subsequently, the chitosan is cross-linked by adding to the emulsion an isocyanate and a tannic acid thereby forming biodegradable core-shell microcapsules. Preferably the chitosan is fungal chitosan, the isocyanate is a polyfunctional isocyanate having at least two isocyanate functional groups and the tannic acid is hydrolyzed tannic acid, unhydrolyzed tannic acid or a combination thereof. In some aspects, the aqueous solution further comprises carrageenan, gum Arabic (gum acacia), or a combination thereof. In other aspects, the polyfunctional isocyanate is a biuret, isocyanurate, allophanate, uretdione, oligomeric hexamethylene diisocyanate, or a combination thereof.

In yet further aspects, wherein: (a) obtained slurry of the biodegradable core-shell microcapsules has a level of self-condensed polyfunctional isocyanate that is below 10%, below 5%, below 3%, below 1% or below 0.5%, relative to total weight of the polyfunctional isocyanate used to form wall of the microcapsules; (b) materials used to form wall of the microcapsules do not form a blend of biodegradable materials and non-biodegradable materials; (c) materials used to form wall of the microcapsules forms a blend of biodegradable materials and non-biodegradable materials, wherein levels of the non-biodegradable materials are below 10%, below 5%, below 3%, below 1% or below 0.5%, relative to weight of the microcapsules; or (d) materials used to form the microcapsules form a blend of biodegradable materials and non-biodegradable materials, wherein the biodegradation rate of all components of the blend is at least 20%, 30%, 40%, 50%, 60%, 65%, 70%, 75%, 80%, 85%, 90%, 95% or 98%, within 60 days according to OECD301F or OECD310.

In yet further aspects, the shell is further cross-linked with an amino acid, preferably the amino acid is selected from the group consisting of lysine, arginine, ornithine, cysteine, serine, threonine, asparagine, glutamine, and a combination thereof, preferably lysine. Preferably, the active material is a fragrance, flavor, agricultural active, pesticide, pharmaceutical active, nutraceutical active, animal nutrition active, food active, microbio active, malodor counteractant, and/or cosmetic active, preferably a fragrance, more preferably a fragrance comprising at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2, and optionally at least one additional fragrance ingredient.

A consumer product including the biodegradable core-shell microcapsules of this invention is also provided, wherein the consumer product is preferably a fabric softener, fabric conditioner, detergent, scent booster, fabric refresher spray, body wash, body soap, shampoo, hair conditioner, body spray, hair refresher spray, hair dye, hair moisturizer, skin moisturizer, hair treatment, skin treatment, antiperspirant, deodorant, insect repellent, candle, surface cleaner, bathroom cleaner, bleach, cat litter, or refresher spray.

In a particular aspect, the invention also provides biodegradable core-shell microcapsules, wherein the shell includes a biopolymer cross-linked with an isocyanate and a hydrolyzed tannic acid and the core includes an active material, wherein the microcapsules shell has a biodegradation rate of at least 60% within 60 days according to OECD301F or OECD 310, and the active material comprises a fragrance, flavor, agricultural active, pesticide, pharmaceutical active, nutraceutical active, animal nutrition active, food active, microbio active, malodor counteractant, and/or cosmetic active, preferably a fragrance, more preferably a fragrance comprising at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2, and optionally at least one additional fragrance ingredient. Ideally, the biopolymer used in accordance with this aspect comprises: (i) a chitosan, pectin, chitosan oligosaccharide, carrageenan, modified guar, modified glucan, gum Arabic (gum acacia), modified gum Arabic (modified gum acacia), alginate, modified starch, modified cellulose, or a combination thereof, preferably the biopolymer is fungal chitosan; or (ii) a protein, gelatin, collagen, glucoamylase, hydrolyzed protein, fermented protein, hydrophobin, enzyme, partially neutralized citric acid ester; or (iii) a combination of (i) and (ii).

All parts, percentages and proportions referred to herein and in the claims are by weight unless otherwise indicated.

The details of one or more aspects of the invention are set forth in the description below. Other features, objects, and advantages of the invention will be apparent to those skilled in the art from the detailed description and the drawings.

### BRIEF DESCRIPTION OF THE DRAWINGS

While the specification concludes with claims particularly pointing out and distinctly claiming the invention, it is believed that the invention will be better understood from the following description of the accompanying figures wherein:
FIG. 1 shows LC/MS analysis of tannic acid hydrolyzed at pH 5.5, 6.5 and 8.5. Relative amounts of different molecular weight species of hydrolyzed tannic acid (HTA) are shown, with greater amounts of gallic acid being produced as a function of increasing pH.
FIG. 2 shows the biodegradation rate of Microcapsule 7.
FIG. 3 shows the biodegradation rate of Microcapsule 10.

### DETAILED DESCRIPTION OF THE INVENTION

In this invention, fragrance microcapsules were prepared using biopolymers, such as chitosan, preferably chitosan is derived from shellfish chitosan or fungal chitosan, and other natural and biodegradable materials, such as carrageenan, gum Arabic, tannic acid, etc. The microcapsules prepared in accordance with the methods described herein have excellent biodegradation profiles, robust stability and/or excellent performance of the systems compared with commercially available products prepared with melamine formaldehyde and polyurea polymers that are not as readily biodegradable.

The biodegradable core-shell microcapsules of this invention have a core including at least one active material, and a shell composed of at least one biopolymer cross-linked with a polyfunctional isocyanate having at least two isocyanate functional groups and a tannic acid, wherein the tannic acid is hydrolyzed tannic acid, unhydrolyzed tannic acid, or a combination thereof.

"Biodegradable" as used herein with respect to a material, such as a microcapsule shell as a whole and/or a biopolymer of the microcapsule shell, has no real or perceived health and/or environmental issues, and is capable of undergoing and/or does undergo physical, chemical, thermal, microbial and/or biological degradation. Ideally, a microcapsule shell and/or biopolymer is deemed "biodegradable" when the microcapsule shell and/or biopolymer passes one or more of the following tests including: a respirometry biodegradation method in aquatic media, available from Organization for Economic Cooperation and Development (OECD), International Organization for Standardization (ISO, www.iso.org) and the American Society for testing and Material (ASTM) tests including, but not limited to OECD 301F or 310 (Ready biodegradation) , OECD 302 (inherent biodegradation), ISO 17556 (solid stimulation studies) , ISO 14851 (fresh water stimulation studies) , ISO 18830 (marine sediment stimulation studies) , OECD 307 (soil stimulation studies) , OECD 308 (sediment stimulation studies) , and OECD 309 (water stimulation studies). Preferably, the microcapsules are readily biodegradable as determined using a respirometry biodegradation method in aquatic media, OECD 301F or OECD 310 test. More preferably, the shell of the microcapsules are biodegradable if the shell has a biodegradation rate of at least 20%, 30%, 40%, 50%, 60%, 65%, 70%, 75%, 80%, 85%, 90%, 95% or 98%, within 60 days according to the respirometry biodegradation method in aquatic media, OECD301F or OECD310 tests, or most preferably a biodegradation rate of at least 60% within 60 days according to OECD301F test.

As used herein, a "core-shell microcapsule," or more generically a "microcapsule" or "capsule," is a substantially spherical structure having a well-defined core and a well-defined envelope or wall. The "core" is composed of any active material or material submitted to microencapsulation. The "wall" is the structure formed by the microencapsulating biopolymer around the active material core being microencapsulated. In general, the wall of the microcapsule is· made of a continuous, polymeric phase with an inner surface and outer surface. The inner surface is in contact with the microcapsule core. The outer surface is in contact with the environment in which the microcapsule resides, *e.g*., a water phase, skin, or hair. Ideally, the wall protects the core against deterioration by oxygen, moisture, light, and effect of other compounds or other factors; limits the losses of volatile core materials; and releases the core material under desired conditions. In this respect, the core-shell microcapsules of this invention provide controlled release and/or diffusional release of the active material. As used herein, "controlled release" refers to retention of the active material in the core until a specified triggering condition occurs. Such triggers include, e.g., friction, swelling, a pH change, an enzyme, a change in temperature, a change in ionic strength, or a combination thereof.

As used in the context of this invention, "biodegradable core-shell microcapsules" or "a biodegradable core-shell microcapsule composition" refers to a slurry or suspension of biodegradable core-shell microcapsules produced in accordance with the methods and examples described herein. The biodegradable core-shell microcapsule composition of this invention may be used directly in a consumer product, washed, coated, dried (e.g., spray-dried) and/or combined with one or more other microcapsule compositions, active materials, and/or carrier materials.

For the purposes of this invention, a "biopolymer" is a polymer obtained from a natural source (*e.g*., a plant, fungus, bacterium, marine or animal) or modified biopolymer thereof. In some aspects, the biopolymer used in the preparation of the microcapsules is water soluble *(i.e.,* water soluble prior to being cross-linked). In other aspects, the biopolymer is a polypeptide, polysaccharide or polyphenolic compound. In certain aspects, the biopolymer of the microcapsule wall is a single type of polymer, *e.g.,* a polypeptide, a polysaccharide or a polyphenolic compound. In other aspects, the biopolymer of the microcapsule wall is a combination of polymers and/or types of polymers, e.g., (a) at least one polypeptide in combination with at least one polysaccharide, (b) at least one polypeptide in combination with at least one polyphenolic compound, (c) at least one polysaccharide in combination with at least one polyphenolic compound, (d) at least one polypeptide in combination with at least one polysaccharide and at least one polyphenolic compound, or (e) two or more polysaccharides in combination with at least one polyphenolic compound.

The term "sustainable" as used herein with respect to a material, such as microcapsule shell and/or active material (e.g., fragrance ingredients), refers to "biobased" material, which are atoms or molecules obtained from biomass, e.g., obtained from materials containing organic carbon of renewable origin. Source of such carbon can be derived from agricultural products, plants, animals, fungi, microorganisms, marine or forestry materials.

In certain aspects, the shell of the biodegradable core-shell microcapsules includes at least one polysaccharide such as a chitosan, pectin, chitosan oligosaccharide, carrageenan, modified guar, modified glucan, gum Arabic (gum acacia), modified gum Arabic (modified gum acacia), modified starch, modified cellulose, or alginate; a polypeptide such as a protein, gelatin, collagen, hydrolyzed protein, fermented protein, hydrophobin, or enzyme; a partially neutralized citric acid ester, or any combination of the above-referenced biopolymers. In some aspects, the shell of the biodegradable core-shell microcapsules includes at least two, three, or four polysaccharides. In particular, the shell may be composed of chitosan and carrageenan and/or gum Arabic (gum acacia).

In particular aspects, the shell of the core-shell microcapsule is composed of chitosan. "Chitosan" is a linear polysaccharide composed of randomly distributed β-(1-4)-linked D-glucosamine (deacetylated unit) and *N*-acetyl-D-glucosamine (acetylated unit). Important characteristics in determining the functionality of chitosan are the degree of deacetylation (%DDA) and molecular weight (M_{w}). Chitosan is technically defined as chitin with more than 60% DDA. Chitosan with higher %DDA possesses more positively charged amine groups when dissolved in solution. In traditionally sourced crustacean-based chitosan production, large volumes of high temperature, caustic solution are required to chemically remove the acetyl groups. Fungal production of chitosan allows for very high %DDA values due to the nature of the fermentation, with the ability to routinely produce a DDA as high as 99%. In preferred aspects, chitosan is obtained from a fungal source or derived from fungal chitin by chemical deacetylation. Exemplary fungal sources that may be used in the preparation of chitosan include, but are not limited to, *Pleurotus ostreatus* and *Aspergillus niger.*

In certain aspects, the chitosan has a degree of deacetylation (DDA) of from 50% to 95%, preferably from 65 % to 90%. In other aspects, the chitosan has a DDA of ≥ 80%. In certain aspects, the chitosan has an average molecular weight of from 500 Da to 1,000,000 Da, preferably from 2,000 Da to 500,000 Da, more preferably from 10,000 Da to 400,000 Da, or most preferably from 50,000 Da to 250,000 Da.

To achieve the desired performance characteristics *(i.e.,* active material retention and controlled release), the biopolymer is cross-linked with one or more cross-linking agents. As used herein, a "cross-link" is a bond, atom, or group linking the chains of atoms in a biopolymer. In accordance with this invention, the biopolymer (e.g., chitosan, carrageenan, and/or gum Arabic) is cross-linked with a polyfunctional isocyanate having at least two isocyanate functional groups and a tannic acid, wherein the tannic acid is hydrolyzed tannic acid, unhydrolyzed tannic acid, or a combination thereof. Optionally, the biopolymer may be further cross-linked with an amino acid, preferably the amino acid is selected from the group consisting of lysine, arginine, ornithine, cysteine, serine, threonine, asparagine, glutamine and a combination thereof, more preferably the amino acid is lysine.

The term "polyisocyanate" as used here is a collective term for compounds containing at least two isocyanate groups in the molecule (this is understood by the person skilled in the art to mean free isocyanate groups of the general structure: R-N=C=O). The simplest form of polyisocyanates are the diisocyanates. These have the general structure O=C=N-R-N=C=O where R typically represents aliphatic, alicyclic and/or aromatic groups. In certain aspects, the polyfunctional isocyanate is an oligomeric polyisocyanate obtained from hexamethylene diisocyanate (HDI), which is a monomeric diisocyanate. In certain aspects, the polyfunctional isocyanate is an oligomeric polyisocyanate especially having a biuret, isocyanurate, allophanate, uretdione and/or oligomeric HDI structure.

It has been found that the inclusion of tannic acid, in particular a hydrolyzed tannic acid, improves the biodegradability of the microcapsules. Accordingly, in some aspects, the biopolymer, preferably chitosan, is cross-linked with a tannic acid, wherein the tannic acid is hydrolyzed tannic acid, unhydrolyzed tannic acid, or a combination thereof. Hydrolyzed tannic acid can be prepared as described herein by subjecting tannic acid to a pH of between 5.0 and 8.5, preferably between 5.5 to 7.0, most preferably between 5.5 to 6.5, for a suitable amount of time to produce significant hydrolysis of the tannic acid to multiple lower molecular weight species. Ideally, the tannic acid is hydrolyzed such that gallic acid constitutes a substantial percentage of the hydrolysis products.

The core of the biodegradable core-shell microcapsules of this invention includes at least one active material. In certain aspects, the microcapsules include at least two, three, four or more active materials in the core. Active materials that may be encapsulated in the core-shell microcapsule of this invention include, but are not limited to, fragrances, flavors, agricultural actives, pesticides, pharmaceutical actives, nutraceutical actives, animal nutrition actives, food actives, microbio actives, malodor counteractants, and/or cosmetic actives, preferably fragrances, more preferably fragrances comprising at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2.

**Table 1 - Ultra High-Impact Fragrance Ingredients**

| **No.** | **Trade Name** | **CAS** |
|---|---|---|
| 1. | ALD C-11 INTRELEVEN (TT) PRG | 143-14-6 |
| 2. | ALD C-6 TOCO | 66-25-1 |
| 3. | AMBER XTREME^{™} | 476332-65-7 647828-16-8 |
| 4. | AMBERMOR EX | 6790-58-5 |
| 5. | AMBERMORKETAL | 57345-19-4 |
| 6. | AMBROCENIDE CRYST | 211299-54-6 |
| 7. | CALONE | 28940-11-6 |
| 8. | CITRYLAL BHT | 90480-35-6 147060-73-9 |
| 9. | CLONAL | 2437-25-4 |
| 10. | CREOSOL #340 | 93-51-6 |
| 11. | CRESYL ACET PARA COEUR | 140-39-6 |
| 12. | DECENAL,CIS-4 | 21662-09-9 |
| 13. | DODECENAL TRANS-2 TOCO | 20407-84-5 |
| 14. | DUPICAL TOCO | 30168-23-1 |
| 15. | DYNASCONE BHT | 56973-85-4 |
| 16. | FLORAL SUPER | 71077-31-1 |
| 17. | FRAGARONE | 16957-70-3 |
| 18. | GALBASCONE ALPHA 95 PRG | 56973-85-4 |
| 19. | GALBASCONE PRG TOCO | 56973-85-4 56973-84-3 |
| 20. | ISO BUTYL QUINOLINE | 67634-06-4 |
| 21. | ISO BUTYL THIAZOLE | 18640-74-9 |
| 22. | JAMUNATE | 7605-52-9 |
| 23. | KOUMALACTONE 10 PCT TEC FIRM | 77-93-0 |
| 24. | MANGONE CSM | 38462-22-5 |
| 25. | MELOZONE | 30772-79-3 |
| 26. | METH HEPTIN CARBONATE | 111-12-6 |
| 27. | METH LAITONE 10 PCT DPG | 110-98-5 |
| 28. | METH PHEN ETH ETHER | 3558-60-9 |
| 29. | NONADIENAL,2-TR-6-CIS TOCO | 557-48-2 |
| 30. | NONENOL,CIS-6 TOCO | 35854-86-5 |
| 31. | OPALENE TOCO | 174155-47-6 |
| 32. | ORENYLE | 29127-83-1 |
| 33. | ORRIS ALD TOCO | 60784-31-8 |
| 34. | OXANE 50 PCT TEC | 59323-76-1 59324-17-3 |
| 35. | PASSION FRUIT CMPD | 59323-76-1 |
| 36. | PHARAONE 10 PCT DPG | 110-98-5 |
| 37. | PYRAZINE 044 (METH) | 109-08-0 |
| 38. | ROSE OXIDE "L" TOCO | 16409-43-1 |
| 39. | ROSYRANE SUPER | 60335-71-9 |
| 40. | SACRAZOLE-018 | 137-00-8 |
| 41. | THIAZOLE (2-ISO PROP 4-METH) | 15679-13-7 |
| 42. | TRIDECENE-2-NITRILE | 22629-49-8 |
| 43. | TROPIC ALIA TOCO | 10138-32-6 |
| 44. | VIONIL NEAT | 67019-89-0 |
| 45. | HEALINGWOOD *¹* | Natural Oil |
| 46. | VIOLET LEAF ABS EGYPT LMR *¹* | Natural Oil |

| | | |
|---|---|---|
| *¹* Available from International Flavors & Fragrances Inc. | | |

**Table 2 - High-Impact Fragrance Ingredients**

| **No.** | **Trade Name** | **CAS** |
|---|---|---|
| 1. | ACETOPHENONE | 98-86-2 |
| 2. | AGRUNITRILE | 51566-62-2 |
| 3. | ALD AA TRIPLAL BHT | 68039-49-6 |
| 4. | ALD C-10 | 112-31-2 |
| 5. | ALD C-11 MOA BHT | 19009-56-4 |
| 6. | ALD C-11 ULENIC TOCO | 112-45-8 |
| 7. | ALD C-11 UNDECYLIC TOCO | 112-44-7 |
| 8. | ALD C-12 LAURIC TOCO | 112-54-9 |
| 9. | ALD C-12 MNA TOCO | 110-41-8 |
| 10. | ALD C-16 STRAWB#2 | 77-83-8 |
| 11. | ALD C-18 | 104-61-0 |
| 12. | ALD C-8 TOCO | 124-13-0 |
| 13. | ALD C-9 TOCO | 124-19-6 |
| 14. | AMBERTONIC | 1392325-86-8 |
| 15. | AMBRETTOLIDE | 28645-51-4 |
| 16. | AQUAFLORA TOCO | 1339119-15-1 |
| 17. | BENZALD FFC | 100-52-7 |
| 18. | CASHMERAN | 33704-61-9 |
| 19. | CEDRAMBER | 67874-81-1 |
| 20. | CITRAL NEW | 5392-40-5 |
| 21. | COOLWOOD | 1340502-69-3 |
| 22. | CRESOL PARA EXTRA | 106-44-5 |
| 23. | CRISTALFIZZ | 1093653-57-6 |
| 24. | CYCLAPROP | 68912-13-0 |
| 25. | CYCLEMAX | 7775-00-0 |
| 26. | DAMASCENONE TOCO | 23696-85-7 |
| 27. | DAMASCONE DELTA | 71048-82-3 |
| 28. | DELPHONE | 4819-67-4 |
| 29. | DIPHEN OXIDE | 101-84-8 |
| 30. | DORIFFOX | 149713-23-5 |
| 31. | ETH ACETO ACET | 141-97-9 |
| 32. | ETH CAPROATE | 123-66-0 |
| 33. | ETH PHEN GLYC | 121-39-1 |
| 34. | ETH SAL | 118-61-6 |
| 35. | ETH-2-METH BUTY | 7452-79-1 |
| 36. | EUCALYPTOL USP | 470-82-6 |
| 37. | FLORHYDRAL TOCO (ELINCS) | 125109-85-5 |
| 38. | FRUITATE (ELINCS) | 129520-41-8 |
| 39. | HEXADECANOLIDE BHT | 109-29-5 |
| 40. | HEXENYL ISOBUTY,CIS-3 (VERDURAL B) | 41519-23-7 |
| 41. | INDOLE | 120-72-9 |
| 42. | IONONE ALPHA TOCO | 127-41-3 |
| 43. | IRONE V BHT | 79-69-6 |
| 44. | ISO CYCLO CITRAL TOCO | 1423-46-7 |
| 45. | LACTONE OF CIS JASMONE TOCO | 70851-61-5 |
| 46. | LEMONILE | 61792-11-8 |
| 47. | MELONAL TOCO | 106-72-9 |
| 48. | MENTHONE 85 | 89-80-5 |
| 49. | METH BENZOATE | 93-58-3 |
| 50. | METH CINNAMATE TOCO | 103-26-4 |
| 51. | METH DH JASMONATE | 24851-98-7 |
| 52. | METH HEPTYL KETONE | 821-55-6 |
| 53. | METH JASMONATE TOCO | 39924-52-2 |
| 54. | METH OCTIN CARBONATE | 111-80-8 |
| 55. | METH PARA CRESOL | 104-93-8 |
| 56. | METH TUBERATE RD | 35205-76-6 |
| 57. | MUSCEMOR (ELINCS) | 82356-51-2 |
| 58. | MYRAC ALD BHT | 52475-89-5 |
| 59. | NECTARYL LRG 1371 ELINCS | 95962-14-4 |
| 60. | OCEANOL | 33662-58-7 |
| 61. | OPERANIDE (ELINCS) | 823178-41-2 |
| 62. | PHEN ETH ACET | 103-45-7 |
| 63. | PINEAPPLE CMPD | 3658-77-3 |
| 64. | PINO ACETALD TOCO | 33885-51-7 |
| 65. | ROSALVA | 13019-22-2 |
| 66. | ROSE OXIDE TOCO | 16409-43-1 |
| 67. | ROSETHYL | 64988-06-3 |
| 68. | SAFRALEINE | 54440-17-4 |
| 69. | SINFONIDE | 1315251-11-6 |
| 70. | STARFLEUR TOCO | 1254940-85-6 |
| 71. | SYLVONIC | 98-55-5 |
| 72. | TERPINOLENE P UB BHT | 586-62-9 |
| 73. | TOFFEE LACTONE 2067 | 705-86-2 706-14-9 |
| 74. | TOFFEETONE FOR NON TSCA USE ONLY | 13537-82-1 |
| 75. | TONKALACTONE | 54491-17-7 |
| 76. | TRIFERNAL BHT | 16251-77-7 |
| 77. | ULTRAVANIL Q COLIPA | 2563-07-7 |
| 78. | UNDECALACTONE,DELTA | 710-04-3 |
| 79. | UNDECAVERTOL TOCO | 81782-77-6 |
| 80. | VANITROPE | 94-86-0 |
| 81. | VARAMOL-106 | 7786-61-0 |
| 82. | VELTOL PLUS | 4940-11-8 |
| 83. | VERAMOSS | 4707-47-5 |
| 84. | VERIDIAN | 811412-48-3 |
| 85. | VERTOLIFF TOCO | 36635-35-5 |
| 86. | VERTONIC | 1945993-03-2 |
| 87. | VETIVER ACET HAITI TOCO BLO | 84082-84-8 |
| 88. | YARA YARA | 93-04-9 |
| 89. | YLANGANATE | 89-71-4 |
| 90. | ARMOISE OIL PURE *¹* | Natural oil |
| 91. | BLKCURRANT BUD ABS BURGUNDY LMR FOR LIFE *¹* | Natural oil |
| 92. | CHAMOMILE OIL ENG *³* | Natural oil |
| 93. | CHAMOMILE OIL ROMAN LMR SFO *¹* | Natural oil |
| 94. | CINNAMON BARK ESSENTIAL LMR *¹* | Natural oil |
| 95. | DAVANA OIL LMR FLG SFO *¹* | Natural oil |
| 96. | EUGENOL NAT EX CLOVE LEAF OIL | Natural oil |
| 97. | JASMIN ABS EGYPT LMR *¹* | Natural oil |
| 98. | ORANGE FLOWER WATER ABS TUNISIA LMR *¹* | Natural oil |
| 99. | OSMANTHUS ABS LMR *¹* | Natural oil |
| 100. | PATCHOULI OIL LIGHT BLO *²* | Natural oil |
| 101. | PEPPER PINK CO2 LMR *¹* | Natural oil |
| 102. | ROSE ABS DAMASCENA PURE BLO *¹* | Natural oil |
| 103. | SINENSAL NATURAL 20 EX ORANGE *³* | Natural oil |
| 104. | THYME OIL WHITE SPAIN BLO *¹* | Natural oil |

| | | |
|---|---|---|
| *¹* Available from International Flavors & Fragrances Inc (New York). *²* Available from TRIPPER PTE Ltd. (Indonesia). *³* Available from TREATT & CO. Ltd. (United Kingdom). | | |

In addition to the fragrance ingredients listed in Tables 1 and 2, the fragrance may include at least one additional fragrance ingredient. Preferably, the fragrance may include 1, 2, 3, 4, 5, 6, 7, 8, 9, 10, 15, 20, 25, 30, 35, 40 or more additional fragrance ingredients, which are not listed in Tables 1 and 2. Non-limiting examples of such additional fragrance ingredients include those described in US 2018/0325786 A1, US 4,534,891, US 5,112,688, and US 5,145,842. Other suitable active materials that can be encapsulated include those listed in WO 2016/049456, pages 38-50.

The additional fragrance ingredients, when combined with one or more fragrance ingredients of Tables 1 and 2, constitute the total fragrance composition. In this respect, the balance of the 100 wt% relative to the total weight of the fragrance component is made up of one or more Ultra High-Impact and High-Impact fragrance ingredients of Tables 1 and 2 and one or more additional fragrance ingredients.

It has been surprisingly discovered that a fragrance that includes certain types of Ultra High-Impact and High-Impact fragrance ingredients results in High Performance fragrance ingredients for use in fabric care composition, preferably fabric conditioner, which improves fragrance profile and/or performance of the system. As used herein, the term "fragrance profile" means the description of how the fragrance is perceived by the human nose at any moment in time. The fragrance profile may change over time. It is a result of the combination of the base, heart and top notes, if present, of a fragrance. Base notes are characterized by providing animalic, woody, sweet, amber or musky characters, and not being very volatile. Heart notes are associated with desirable characters such as floral characters (e.g., jasmine, rose), fruity, marine, aromatic or spicy characters. The "top or head notes" provide citrusy, green, light, or fresh characters and tend to evaporate quickly due to their high volatility. A fragrance profile is composed of two characteristics: 'intensity' and 'character'. The 'intensity' relates to the perceived strength whilst 'character' refers to the odor impression or quality of the perfume, *i.e.,* fresh, clean, *etc.*

In addition to the active materials, the present invention also contemplates the incorporation of additional components including solvents and core modifier materials in the core encapsulated by the microcapsule wall. Other components include solubility modifiers, density modifiers, stabilizers, viscosity modifiers, pH modifiers, deposition aids, capsule formation aids, catalysts, processing aids or any combination thereof. These components can be present in the wall or core of the capsules, or outside the capsules in the microcapsule composition to improve solubility, stability, deposition, capsule formation, and the like. Further, the additional components may be added after and/or during the preparation of the microcapsule composition of this invention. Suitable solvents or dispersants include, e.g., isopropanol, ethyl acetate, acetic acid, caprylic/capric triglyceride, polyoxyethylated castor oil, anionic, nonionic and cationic block polymers, and the like, or any combination thereof.

Suitable solvents for dispersing isocyanate include ethyl acetate, 3-methoxybutyl acetate, diethylene glycol monoethyl ether acetate (DGMEA), propylene glycol diacetate, diethyl malonate, triacetin, triethyl citrate, ethyl acetoacetate, benzyl acetone, butyl carbitol acetate, and the like, or any combination thereof.

In some aspects, the wall material of the core-shell microcapsule is composed of between 0.2% to 11.0%, preferably between 1.2% to 4.5% by weight biopolymer; between 0.1% and 10%, preferably between 0.5% and 5% by weight cross-linker; between 0.5% and 1.0%, preferably between 0.1% and 0.5% by weight dispersant; between 10.0% to 90.0%, preferably between 15.0% and 40.0% by weight active material; between 5.0% and 90.0%, preferably between 10.0% and 50.0% by weight core modifier; and optionally between 0.01% and 0.5%, preferably between 0.05% and 0.2% by weight rheology modifier.

In certain aspects, the wall material of the core-shell microcapsule is composed of at least one, two, or three biopolymers. In certain aspects, the wall material is composed of between 0 % to 5.0%, preferably between 0.2% to 2.0% carrageenan; between 0.1% to 3.5%, preferably between 0.5% to 3.0% gum Arabic (gum acacia); and/or between 0.1% to 3.0%, preferably between 0.5% to 1.5% chitosan by dry weight of the microcapsule.

In certain aspects, one or a combination of two, three, or four cross-linkers is used. In particular, the cross-linking component of the microcapsule includes between 0.01% and 2.0%, preferably between 0.05% and 0.5% polyisocyanate; between 0.0% and 10%, preferably between 0.1% and 5.0% tannic acid; between 0.1% and 10%, preferably between 0.2% and 5.0% hydrolyzed tannic acid; and/or between 0.0% and 1.8%, preferably between 0.0% and 1.0% L-lysine by weight of the microcapsule.

The active material component (e.g., fragrance) of the core-shell microcapsules is preferably present at between 5.0% to 90.0%, preferably between 10.0% and 40.0% by weight of the microcapsule. When the fragrance includes a combination of the Ultra High-Impact fragrance ingredients and the High-Impact fragrance ingredients listed in Tables 1 and 2, the fragrance can be used in an aqueous fabric conditioner product at a significantly reduced dosage (*e.g.,* at least 2-, 5- to 10-fold lower levels) as compared to a standard fragrance that does not include the Ultra High-Impact fragrance ingredients and High-Impact fragrance ingredients listed in Tables 1 and 2.

In particular, the fragrance of this invention can be used at a dosage level of ≤ 1 wt% relative to the total weight of the microcapsule composition without significantly impacting the fragrance profile, i.e., perceived fragrance intensity, perceived fragrance longevity and/or perceived fragrance fidelity, particularly for select characters (e.g., fresh and/or clean). In some aspects, the fragrance is used at a dosage level of less than or equal to 1 wt%, 0.99 wt%, 0.95 wt%, 0.9 wt%, 0.8 wt%, 0.7 wt%, 0.6 wt%, 0.5 wt%, 0.4 wt%, 0.3 wt%, 0.2 wt%, 0.1 wt%, 0.05 wt% or 0.01 wt% of the total weight of the aqueous fabric conditioner composition, or any range delimited by any pair of the foregoing values.

Additional components include between 0.1% and 1.0%, preferably between 0.1% and 0.3% dispersant such as polyoxyethylated castor oil; between 5.0% and 90.0%, preferably between 10% and 50.0% core modifier such as caprylic/capric triglyceride; and optionally between 0.05% and 1.0%, preferably between 0.1% and 0.5%, rheology modifier such as xanthan gum, by weight of the microcapsule.

The microcapsules of this invention each have a size (in diameter) in the range of 0.1 micron to 1000 microns (e.g., 0.5 micron to 500 microns, 1 micron to 200 microns, 1 micron to 100 microns, and 1 micron to 50 micron) with a lower limit of 0.1 micron, 0.5 micron, 1 micron, 2 microns, 5 microns and 20 microns, and an upper limit of 1000 microns, 500 microns, 200 microns, 100 microns, 75 microns, 50 microns, 30 microns, 20 microns, 10 microns, and 5 microns.

Another aspect of the present invention is the desire to move toward the use of fragrance ingredients and/or microcapsules derived from "Green Chemistry" principles. Green Chemistry is focused on the design of products and processes that minimize environmental impact, particularly by using renewable feedstocks. In other words, the raw material or feedstock used to make the fragrance ingredients and/or microcapsules should be sustainable rather than depleting whenever technically and economically practicable. Preferably, the fragrance component and/or microcapsules of the present invention has a bio-renewable carbon (BRC) content of at least 50%, at least 55%, at least 60%, at least 65%, at least 70%, at least 75%, at least 80%, at least 85%, at least 90% or at least 95%. As used herein, "BRC" refers to carbon that is part of the earth's natural environment and non-fossil-based carbon. BRC are naturally occurring renewable, repurposed and/or upcycled carbon resources that can be replenished to replace the portion depleted by usage and consumption, either through natural reproduction, or other recurring processes in a finite amount of time (such as within a human lifetime). BRC would exclude carbon that comes from virgin crude oil.

In some aspects, one or more non-confined or unencapsulated active materials can also be included post-curing. Such active materials may be the same or different than the encapsulated active material and may be included at a level of from 0.01% to 20%, or preferably from 2% to 10% by weight of the microcapsule composition *(i.e.,* microcapsule slurry).

The microcapsules of this invention can also be combined with one or more other delivery systems such as polymer-assisted delivery compositions (US 8,187,580), fiber-assisted delivery compositions (US 2010/0305021), cyclodextrin host-guest complexes (US 6,287,603 and US 2002/0019369), pro-fragrances (WO 2000/072816 and EP 0 922 084), and any combination thereof. More exemplary delivery systems that can be incorporated are coacervate capsules, cyclodextrin delivery systems, and pro-perfumes.

Furthermore, microcapsules having one or more different characteristics can be combined to provide desirable or tailored release profiles and/or stability. In particular, the microcapsule composition can include a combination of two or more types of microcapsules that differ in their encapsulating wall materials, microcapsule size, amounts of wall materials, the thickness of the wall, the degree of polymerization, the degree of crosslinking, ratios between the wall materials and the active material, core modifiers, scavengers, active materials, cure temperatures, heating rates during the curing, curing times, the rupture force or fracture strength, or a combination thereof. In some aspects, the microcapsule composition is composed of two, three, four, five, six, seven or more different types of capsules that differ by one or more of the above-referenced characteristics.

When assessing storage stability, fragrance retention within the microcapsule may be measured directly after storage, at a desired temperature and time periods such as four weeks, six weeks, two months, three months or more in a consumer product base. The preferred manner is to measure total headspace of the consumer product at the specified time and to compare the results to the headspace of a control consumer product made to represent 0% retention via direct addition of the total amount of fragrance present. Alternatively, the consumer product may be performance tested after the storage period and the performance compared to the fresh product, either analytically or by sensory evaluation. This measurement often involves either measuring the fragrance headspace over a substrate used with the product, or odor evaluation of the same substrate. In certain aspects, retention of the active material in the core of the instant microcapsules is assessed in a consumer product base, *e.g.* , under storage conditions such as at a temperature in the range of 25°C to 40°C, or more preferably in the range of 30°C to 37°C, or most preferably 37°C, for an extended period of time of at least 2 weeks, 4 weeks, 6 weeks, 8 weeks, 16 weeks, or 32 weeks. In certain aspects, the microcapsules of this invention retain at least 30% of the active material when added to a consumer product base. In particular aspects, the microcapsules of this invention, when added to a consumer product base, retain between at least 40% and 90% of the active material after being stored at 37°C for at least 4 weeks, 8 weeks or 12 weeks.

In general, the biodegradable core-shell microcapsules of this invention are prepared by emulsifying (i) an aqueous solution including chitosan with (ii) an oil phase including an active material to form an emulsion; and (b) cross-linking the chitosan by adding to the emulsion an isocyanate and a tannic acid thereby forming biodegradable core-shell microcapsules. In certain aspects, the chitosan is fungal chitosan, the isocyanate is a polyfunctional isocyanate having at least two isocyanate functional groups (*e.g.,* a biuret, isocyanurate, allophanate, uretdione, oligomeric hexamethylene diisocyanate, or a combination thereof), and the tannic acid is hydrolyzed tannic acid, unhydrolyzed tannic acid, or a combination thereof. In other aspects, the aqueous solution optionally includes carrageenan, gum Arabic (gum acacia), or a combination thereof and/or the shell is further optionally cross-linked with an amino acid, preferably selected from the group consisting of lysine, arginine, ornithine, cysteine, serine, threonine, asparagine, glutamine, and a combination thereof, preferably lysine. Moreover, the method may further include a step of curing the microcapsules. As used herein, "curing" refers to a toughening or hardening process of a polymer by cross-linking of polymer chains, brought about by heat, chemical additives, or light radiation. In some aspects, the microcapsules are cured at elevated temperatures, e.g., between 35°C to 150°C, preferably between 55°C to 90°C for between 1 and 5 hours.

The method may alternatively or further include a step of drying the microcapsules to remove water. The drying temperature is in the range of 20°C and 250°C, or at room temperature. In some aspects, the microcapsules are dried by a dehumidifier configured to supply desiccated air to the microcapsules, a radiant heat source for facilitating drying of the microcapsules or submitting the microcapsules under a gas flow to obtain dried free-flowing microcapsules. It is understood that any standard method known by a person skilled in the art to perform such drying is also applicable.

Compared to conventional polyisocyanate and biodegradable polymer microcapsules, the walls of the present microcapsules is formed from chitosan cross-linked with a polyfunctional isocyanate having at least two isocyanate functional groups and a tannic acid, with the polyfuctional isocyanate being added in such a way to thereby substantively or completely eliminating self-condensation of polyisocyanate. Preferably, the biodegradable core-shell microcapsules of this invention are substantively or completely free of self-condensation of polyisocyanate and therefore exhibit superior (or 'true') biodegradability properties *(i.e.,* by reducing or avoiding blends of biodegradable and non-biodegradable materials). In particular, the obtained slurry of biodegradable core-shell microcapsules has a level of self-condensation of polyfunctional isocyanate *(i.e.,* polyurea and/or polyurethane) that is below 10%, below 5%, below 3%, below 1% or below 0.5%, relative to total weight of the polyfunctional isocyanate used to form wall of the microcapsules.

The biodegradable core-shell microcapsule composition of this invention is well-suited for inclusion in any of a variety of consumer products where controlled release of active materials (e.g., fragrances or flavors) is desired. The microcapsule composition of this invention can be added to a consumer product base directly or be printed onto a product base or a movable product conveyor (e.g., a non-stick belt) for drying. See WO 2019/212896 A1. The biodegradable core-shell microcapsules can be added to the consumer product base at a level in the range of 0.001% to 50%, or more preferably 0.01% to 50% by weight of the consumer product base. In particular, the biodegradable core-shell microcapsules are suitably included in a consumer product such as a fabric softener, fabric conditioner, detergent, scent booster, fabric refresher spray, body wash, body soap, shampoo, hair conditioner, body spray, hair refresher spray, hair dye, hair moisturizer, skin moisturizer, hair treatment, antiperspirant, deodorant, skin treatment, insect repellant, candle, surface cleaner, bathroom cleaner, bleach, cat litter, or refresher spray.

The values and dimensions disclosed herein are not to be understood as being strictly limited to the exact numerical values recited. Instead, unless otherwise specified, each such value is intended to mean both the recited value and a functionally equivalent range surrounding that value. For example, a value disclosed as "50%" is intended to mean "about 50%." Moreover, ranges include those provided as well as any range delimited by any pair of the recited values. For example, a range disclosed as 0.5% to 5.0% encompasses a range of 0.5% to 1.0%, 0.8% to 3.0%, 1.0% to 5.0%, etc.

### EXAMPLES

The following non-limiting examples are provided to further illustrate the present invention and are not to be construed as limitations of the invention, as many variations of the present invention are possible without departing from its spirit or scope.

### Example 1: Materials and Methods

*Sample Preparation for Biodegradability Evaluation.* To ensure a clear and accurate biodegradability evaluation, the microcapsules were subjected to a vigorous cleaning process to purify the microcapsule shells before the biodegradability evaluation. The objective of the cleaning process was to remove materials that were not part of the microcapsule shells and/or were not tightly associated with the microcapsule shells.

To clean the microcapsules, 300 grams (g) of microcapsule slurry was washed at least three times with water, and then centrifuged at 6000 rpm for 15 minutes (Sorvall LYNX 6000 Superspeed Centrifuge). Fragrance capsules formed a layer of cake and settled on the topmost layer after centrifugation. The aqueous layer and the precipitation layer were pumped out by a peristatic pump at 300 rpm (MASTERFLEX^{®} LIS). The water wash was repeated twice more, and the damp capsule cake was collected and freeze dried (Millrock Technology, Lab Series Dryer, Model LD53) to remove water. Dry fragrance microcapsules were subject to methanol extraction at 65°C (50 g dry capsule in 300 mL methanol, BUCHI^{®} Universal Extractor E-800) multiple times until the residual fragrance in the purified walls was less than 2% (w/w). The cleaned capsule walls were vacuum dried at 30°C until fully dried and were then used in the biodegradability evaluation set forth under OECD301F/OECD310.

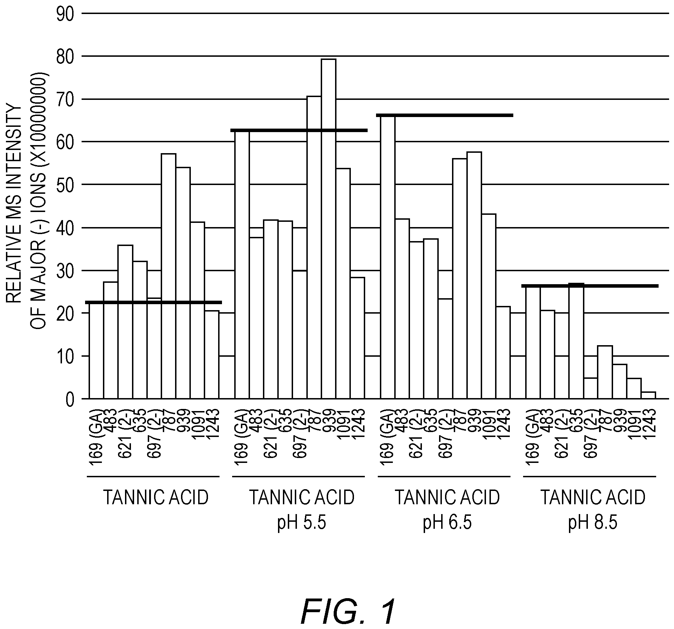

*Preparation Hydrolyzed Tannic Acid (HTA).* A 30% tannic acid solution was adjusted to pH 5.5, 6.0, 6.5, 7.5 and 8.5 with an aqueous NaOH solution (5 to 25 wt/wt. %). The treated solution was stirred at 300 rpm for 18 hours at room temperature. Optionally, the hydrolysis reaction was quenched by adjusting the pH to 4.0 using 30% citric acid solution. LC/MS analysis demonstrated significant hydrolysis of tannic acid to multiple lower molecular weight species, with greater amounts of gallic acid produced as a function of increasing pH (FIG. 1). Tannic acid hydrolyzed at pH 5.5/6.0 was used in the preparation of exemplary Microcapsules 9 to 13.

*General Process of Preparing Microcapsules.* Biodegradable microcapsules were produced by preparing an aqueous phase by dissolving the wall forming biomaterials in water; preparing an oil phase containing core material and a core modifier; forming an emulsion by mixing the aqueous phase and the oil phase under shear; adding to the emulsion a solution containing an isocyanate dissolved in an organic solvent to form a slurry; adding to the slurry a hydrolyzed tannic acid and a lysine solution and curing the microcapsule at a temperature of 35°C to 150°C. Unless otherwise indicated, all percentages are weight-based percentages.

### Example 2: Chitosan/Tannic Acid Microcapsules (Microcapsule 1)

An oil phase was first prepared by mixing 102 g of Eden (fragrance commercially available from International Flavors & Fragrances Inc., NY) and 11.34 g of caprylic/capric triglyceride (a core solvent, commercially available under the tradename NEOBEE^{®} oil M-5; Stepan, Chicago, IL). In a separate beaker, 457.11 g of an aqueous solution was obtained by dissolving fungal chitosan (9.0 g), gum acacia (6.0 g), and a polyoxyethylated castor oil (1.8 g, a capsule formation aid commercially available under the tradename TOXIMUL^{®} 8240; Stepan, Chicago, IL) in water. The oil phase was then emulsified into the aqueous phase to form an oil-in-water emulsion under a shearing rate of 8600 rpm for three minutes.

The oil-in-water emulsion was prepared under constant mixing at room temperature. An aliphatic polyisocyanate (1.2 g) (a polyisocyanate based on hexamethylene diisocyanate (HDI) commercially available under the tradename DESMODUR^{®} N100A; Bayer, Leverkusen, Germany) in ethyl acetate (4.5 g) (Sigma-Aldrich, St. Louis, MO) solution was added to the emulsion. The mixture was cured at room temperature for 30 minutes. Then, the mixture was cured at 60°C for 1 hour. Subsequently, 10.8 g of 30% tannic acid aqueous solution (Tanal-02; Ajinomoto, Japan) was added under constant mixing. The slurry was cured for 2 hours at 60°C. L-lysine (4.131g of a 15% solution; Sigma-Aldrich, St. Louis, MO) was added and the slurry was cured for an additional 1 hour to obtain Microcapsule 1 dispersed in the aqueous phase.

### Example 3: Chitosan/Carrageenan Microcapsules (Microcapsule 2)

An oil phase was first prepared by mixing 102 g of a model fragrance and 11.34 g of caprylic/capric triglyceride (a core solvent, commercially available under the tradename NEOBEE^{®} oil M-5; Stepan, Chicago, IL). In a separate beaker, 457.11 g of an aqueous solution was obtained by dissolving fungal chitosan (9.0 g), gum acacia (a capsule formation aid, 6.0 g), Carrageenan A-CAT (IFF Nutrition and Biosciences, Wilmington DE) (6.0 g), and a polyoxyethylated castor oil (1.8 g, commercially available as TOXIMUL^{®} 8240; Stepan, Chicago, IL) in water. The oil phase was then emulsified into the aqueous phase to form an oil-in-water emulsion under a shearing rate of 8600 rpm for three minutes.

The oil-in-water emulsion was prepared under constant mixing at room temperature. An aliphatic polyisocyanate (1.2 g) (a polyisocyanate based on HDI commercially available as DESMODUR^{®} N100A; Bayer, Leverkusen, Germany) in ethyl acetate (4.5 g) (Sigma-Aldrich, St. Louis, MO) solution was added to the emulsion. The mixture was cured at room temperature for 30 minutes. Subsequently, 6.0 g of a 30% tannic acid aqueous solution (Tanal-02; Ajinomoto, Japan) was added under constant mixing for 30 minutes. Lysine (9.3 g of a 15% solution; Sigma-Aldrich, St. Louis, MO) was added and the mixture was incubated for 30 minutes. The mixture was then heated to 60°C. Two portions of 6.0 g of 30% tannic acid aqueous solution (same as above) and two portions of 9.3 g of 15% L-Lysine (same as above) were added into the mixture, alternatively; with 30 minutes of curing with each successive addition. In total, 18.0 grams of tannic acid and 27.9 grams of Lysine solutions were added over a 2-hour period. The resulting slurry containing Microcapsule 2 was dispersed in the aqueous phase.

### Example 4: Chitosan/Carrageenan Microcapsules (Microcapsule 3)

Microcapsule 3 was prepared following the procedure described in Example 3 except that after 2 hours curing time at 60°C, one extra hour curing at 80°C was added.

### Example 5: Chitosan/Carrageenan Microcapsules (Microcapsules 4-6)

Microcapsules 4-6 were prepared by the same process described in Example 3 using the formulations provided in Table 3.

**Table 3**

| **Ingredients (%)** | **Microcapsule 4** | **Microcapsule 5** | **Microcapsule 6** |
|---|---|---|---|
| Fungal Chitosan | 1.5 | 1.5 | 1.5 |
| Carrageenan | 1.0 | 1.0 | 1.0 |
| Gum Acacia | 1.0 | 1.0 | 1.0 |
| TOXIMUL^{®} 8240 | 0.3 | 0.3 | 0.3 |
| DESMODUR^{®} N100A | 0.2 | 0.1 | 0.05 |
| Tannic Acid | 0.9 | 0.9 | 0.9 |
| Lysine | 0.7 | 0.7 | 0.7 |
| Fragrance | 17.0 | 17.0 | 17.0 |
| NEOBEE^{®} | 1.9 | 1.9 | 1.9 |

### Example 6: Chitosan/Carrageenan/HTA Microcapsules (Microcapsules 7-9)

Microcapsules 7-9 were prepared following the procedure described in Example 3 except that 9.0 g or 18.0 g HTA solution was used instead of un-treated tannic acid solution. The formulations for Microcapsules 7-9 are provided in Table 4.

**Table 4**

| **Ingredients (%)** | **Microcapsule 7** | **Microcapsule 8** | **Microcapsule 9** |
|---|---|---|---|
| Fungal Chitosan | 1.5 | 1.5 | 1.5 |
| Carrageenan | 1.0 | 1.0 | 1.0 |
| Gum Acacia | 1.0 | 1.0 | 1.0 |
| TOXIMUL^{®} 8240 | 0.3 | 0.3 | 0.3 |
| DESMODUR^{®} N100A | 0.2 | 0.15 | 0.1 |
| HTA | 0.5 | 0.5 | 0.9 |
| Lysine | 0.7 | 0.35 | 0.7 |
| Fragrance | 17.0 | 17.0 | 17.0 |
| NEOBEE^{®} | 1.9 | 1.9 | 1.9 |

### Example 7: Chitosan/HTA Microcapsules (Microcapsules 10-13)

Microcapsules containing chitosan and hydrolyzed tannic acid (HTA), in the absence of carrageenan, were prepared using the same process as in Example 3, except that a total of 39 grams of HTA solution was added in three equal portions. The compositions of the Microcapsules 10 to 13 are provided in Table 5.

**Table 5**

| **Ingredients (%)** | **Microcapsule** | | | |
|---|---|---|---|---|
| | **10** | **11** | **12** | **13** |
| Fungal Chitosan | 1.5 | 1.5 | 1.5 | 1.8 |
| Gum Arabic | 1.5 | 1.5 | 2.0 | 1.8 |
| TOXIMUL^{®} 8240 | 0.3 | 0.3 | 0.3 | 0.3 |
| DESMODUR^{®} N100A | 0.20 | 0.15 | 0.3 | 0.20 |
| HTA | 0.5 | 0.5 | 0.5 | 0.5 |
| Lysine | 0.7 | 0.7 | 0.7 | 0.7 |
| Fragrance | 17.0 | 17.0 | 17.0 | 17.0 |
| NEOBEE^{®} | 1.9 | 1.9 | 1.9 | 1.9 |

### Example 8: Microcapsules from Water-in-Oil-in-Water (W/O/W)

All solutions are prepared off-line and heated to 50°C prior to processing. Water-in-oil emulsions were prepared by adding the aqueous phase to the oil phase and shearing using an Ultraturax mixer at 10000 rpm for 1 minute. A carrageenan/oil emulsion (emulsion A) was prepared by mixing 500 g of a 3% wt. solution of K Carrageenan (water phase A) with 250 g of Eden/MO/PGPR (80/17/2.5%) (Oil phase) at 50°C and shearing at 10000 rpm for 1 minute, where Eden is a fragrance oil and MO is mineral oil (primol), and PGPR is PG Polyriccinoleate. A tannic acid/oil emulsion (emulsion B) was prepared by mixing 83.3 g of 20% tannic acid (water phase B) with 167 g of Eden/MO/PGPR (80/17.5/2.5% wt.) (Oil phase) at 50°C and shearing at 10000 rpm for 1 minute. A final W/O emulsion (emulsion C) was prepared by mixing emulsion A and emulsion B and gently stirring.

Microcapsules were prepared by adding 1 kg of 3 wt% chitosan solution (W2) to emulsion C and allowing the chitosan solution to sink to the bottom. To the top of the oil phase was added 3.2 grams of a DESMODUR^{®} N100A (Bayer, Leverkusen, Germany) solution (75% DESMODUR^{®} 100N/25% cymene) The mixture was gently stirred. Subsequently, the W/O/W was sheared at 7000 rpm for 1 minute using an Ultraturax. The WOW system was transferred to an overhead mixer, stirred at 500 rpm, and heated to 80°C. The temperature was maintained at 80°C for 1.5 hour. The heat was subsequently removed, and the slurry was stirred at 500 rpm for 5 hours.

### Example 9: Microcapsule Performance

To evaluate fragrance profile performance, microcapsule slurries were blended into a model fabric conditioner base (19% active level) as shown in Table 6.

**Table 6**

| **Material** | **Amount (g)** |
|---|---|
| REWOQUAT^{®} WE 18 (Esterquat; Evonik, 90% in IPA) | 23.46 |
| PROXEL^{®} GLX (1,2-benzisothiazolin-3-one) | 0.11 |
| Water | 76.1 |
| Calcium Chloride (25%) | 0.33 |

The fragrance load was 0.2% neat oil equivalent (NOE). The fragrance intensity of the perfumes encapsulated by the microcapsules was evaluated by conducting a laundry experiment using accepted experimental protocols using European wash machine (Miele). Terry towels were used for the washing experiments and were washed with European fabric conditioners containing fragrance loaded capsules. Washed samples were removed from the washing machine and line dried overnight. The samples were evaluated by a panel of 12 trained judges at three different stages and rated on a scale ranging from 0 to 30.

"Pre-rub" refers to the evaluations of the towels by panelists before the folding of the towels. "Gentle handling" refers to the folding of the towels twice, followed by the evaluation of the towels by the panelists. "Post-rub" refers to vigorous application of mechanical force using both hands to rub the cloths at least once to rupture the capsules and then evaluated for signs of released fragrance. A numerical value of 0 indicates that the fabric produced no signs of released fragrance, 5 indicates that the fabric only produced weak intensity and 30 indicates a very strong smell of released fragrance from the microcapsules. The results presented in Tables 7 and 8 represent results obtained with microcapsules encapsulating two different fragrances, Eden fragrance and Mermaid fragrance, respectively.

**Table 7**

| **Eden Samples** | **Pre-rub intensity** | **Gentle handling intensity** | **Post-rub intensity** |
|---|---|---|---|
| Microcapsule 7 | 8.5 | 11.1 | 12.7 |
| Neat oil | 0 | 1 | 1 |

**Table 8**

| **Mermaid Samples** | **Pre-rub intensity** | **Gentle handling intensity** | **Post-rub intensity** |
|---|---|---|---|
| Microcapsule 10 | 7.97 | 10.53 | 13.60 |
| Neat oil | 2 | 2 | 2 |

These results indicated that biodegradable microcapsules prepared in accordance with the methods and biopolymers herein have superior performance as compared to neat fragrances.

### Example 10: Biodegradation of Chitosan Microcapsules

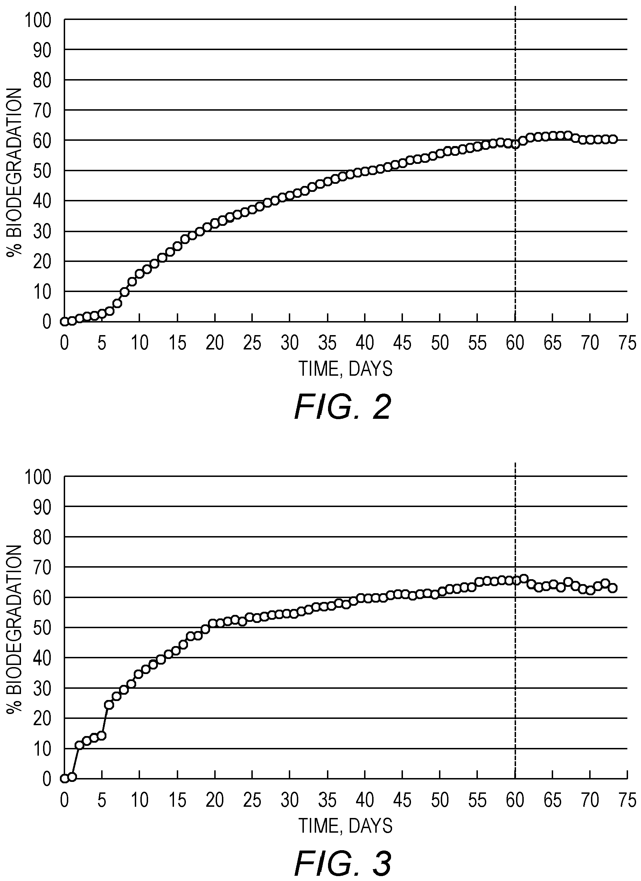

Microcapsules 7 and 10 were washed and evaluated per protocols described by OECD301F. The capsules were found to reach > 60% biodegradation within 60 days. The graphs of the results for Microcapsules 7 and 10 are given in FIG. 2 and FIG. 3, respectively. As a comparison, the biodegradation of MF capsules was also assessed. The results demonstrated that MF capsules only have a biodegradation rate of less than 10% using the same test protocols (data not shown). It was also observed that Microcapsules 7 and 10 prevented a blend of biodegradable and non-biodegradable materials by analysis of the walls of the capsules.

### Example 11: Encapsulated High Performance Fragrance Compositions

High Performance Fragrance Examples 1 and 2 are provided in Table 9 and represent formulations of fragrances comprising encapsulated High Performance fragrance ingredients according to the present invention. Comparative Fragrance Example 1 is provided in Table 9, which represents encapsulated standard fragrance not intended to form the High Performance Fragrance of the present invention. The following fragrance formulations are made by mixing the listed ingredients in the listed proportions in Table 9 at room temperature. The capsules used are Microcapsules 7 and 10.

**Table 9**

| **Ingredient Name** | **CAS Number** | **Comparative Fragrance Example 1** | **High Performance Fragrance Example 1** | **High Performance Fragrance Example 2** |
|---|---|---|---|---|
| ADOXAL TOCO | 141-13-9 | 14.29 | 35.00 | 0.00 |
| ALD AA TRIPLAL TOCO | 68039-49-6 | 28.57 | 60.00 | 65.00 |
| ALD C-10 TOCO | 112-31-2 | 0.00 | 0.00 | 75.00 |
| ALD C-12 MNA TOCO | 110-41-8 | 0.00 | 0.00 | 120.00 |
| ALLYL AMYL GLYCOLATE BHT | 67634-00-8 | 28.57 | 0.00 | 0.00 |
| AMBER XTREME TM | 476332-65-7, 647828-16-8 | 8.57 | 25.00 | 6.00 |
| AMBERTONIC | 1392325-86-8 | 0.00 | 0.00 | 15.00 |
| CASHMERAN | 33704-61-9 | 0.00 | 0.00 | 45.00 |
| CYCLACET | 2500-83-6 | 285.71 | 75.00 | 0.00 |
| CYCLAPROP | 68912-13-0 | 142.86 | 184.50 | 0.00 |
| CYCLOHEXYL SAL (ELINCS) | 25485-88-5 | 0.00 | 0.00 | 21.00 |
| DAMASCONE DELTA TOCO | 71048-82-3 | 0.00 | 0.00 | 50.00 |
| DIHYDRO TERPINEOL | 498-81-7 | 4.06 | 0.00 | 0.00 |
| DIHYDRO TERPINYL ACET | 58985-18-5 | 94.29 | 0.00 | 0.00 |
| EUCALYPTOL USP CSM | 470-82-6 | 0.00 | 0.00 | 100.00 |
| FRUCTALATE (ELINCS) | 72903-27-6 | 0.00 | 0.00 | 25.00 |
| GALBANUM OIL LMR *¹* | natural oil | 0.00 | 0.40 | 0.50 |
| GALBASCONE ALPHA 95 PRG TOCO | 56973-85-4 | 0.00 | 10.00 | 4.00 |
| GALBASCONE PRG TOCO | 56973-85-4, 56973-84-3 | 22.86 | 50.00 | 15.00 |
| GERANIOL 980 PURE | 106-24-1 | 0.00 | 0.00 | 15.00 |
| HERBALIME | 8006-64-2 | 0.00 | 0.00 | 40.00 |
| HEXYL CINN ALD TBHQ | 101-86-0 | 0.00 | 5.10 | 0.00 |
| ISO BORNYL ACET | 125-12-2 | 182.86 | 80.00 | 50.00 |
| JAVAMOR TT (ELINCS) | 198404-98-7 | 0.00 | 0.00 | 4.00 |
| MANZANATE | 39255-32-8 | 0.00 | 0.00 | 35.00 |
| MELONAL TOCO | 106-72-9 | 0.00 | 0.00 | 30.00 |
| METH DH JASMONATE | 24851-98-7 | 0.00 | 35.00 | 0.00 |
| METH IONONE GAMMA "FF" TOCO | 127-51-5 | 0.00 | 0.00 | 150.00 |
| METH IONONE GAMMA A TOCO | 127-51-5 | 0.00 | 40.00 | 0.00 |
| METH PHEN ETH ETHER | 3558-60-9 | 0.00 | 0.00 | 3.00 |
| MONTAVERDI (ELINCS) | 188570-78-7 | 0.00 | 0.00 | 7.50 |
| ORANGE FLOWER ETHER TOCO | 14576-08-0 | 85.71 | 85.00 | 0.00 |
| PAMPLEZEST | 1465004-85-6 | 0.00 | 0.00 | 15.00 |
| PINO ACETALD TOCO | 33885-51-7 | 0.00 | 0.00 | 40.00 |
| ROSYRANE SUPER | 60335-71-9 | 0.00 | 0.00 | 30.00 |
| TERPINOLENE P UB TOCO | 586-62-9 | 57.14 | 130.00 | 0.00 |
| TETRAHYDRO LINALOOL | 78-69-3 | 22.76 | 70.00 | 39.00 |
| TETRAHYDRO LINALYL ACET | 68480-08-0 | 0.00 | 55.00 | 0.00 |
| TETRAHYDRO MYRCENOL | 18479-57-7 | 21.76 | 60.00 | 0.00 |
| | Total | 1000 | 1000 | 1000 |

### Example 12: Fragrance Performance with Encapsulated High Performance Fragrances

The aim of this example is to show the fragrance performance benefit of using encapsulated High Performance fragrances in the fabric conditioner product. A fabric conditioner composition comprising fragrance compositions in Table 8 is washed with laundry and is evaluated by 12 trained panelists as described in Example 9. The results from the sensory panelists are then averaged.

The addition of encapsulated fragrance composition comprising High Performance fragrance ingredients of the present invention into a fabric conditioner base *(i.e.,* High Performance Fragrance Examples 1 & 2) improves fragrance intensity at dry pre and dry gentle handling stages when compared to a benchmark encapsulated fragrance composition *(i.e.,* Comparative Fragrance Example 1). Therefore, when using an encapsulated High Performance Fragrance according to the present invention, fragrance intensity perception at dry pre and dry gentle handling stages can be further improved.

### Example 13: Exemplary Product Compositions

Composition A is an example of fine fragrance composition according to the present invention. It is prepared by admixture of the components described in Table 10, in the proportions indicated.

**Table 10**

| **Ingredient** | **Composition A (wt%*¹*)** |
|---|---|
| Ethanol SD-40 | 60-80 |
| Microcapsules *²* | 2.5-25 |
| DI-Water | Qs |
| Total | 100 |

| | |
|---|---|
| *¹* wt% relative to the total weight of the composition. *²* Encapsulated amount depends on the fragrance loading to 0.3% NOE. | |

Composition B is an example of fabric conditioner composition according to the present invention. It is prepared by admixture of the components described in Table 11, in the proportions indicated.

**Table 11**

| **Ingredient** | **Composition B (wt%*¹*)** |
|---|---|
| Platform 12 fabric conditioner base | 90 |
| Microcapsules *²* | 0.5 - 3.0 |
| DI-Water | Qs |
| Total | 100 |

| | |
|---|---|
| *¹* wt% relative to the total weight of the composition. *²* Encapsulated amount depends on the fragrance loading to 0.2% NOE. | |

Composition C is an example of liquid detergent composition according to the present invention. It is prepared by admixture of the components described in Table 12, in the proportions indicated.

**Table 12**

| **Ingredient** | **Composition C (wt%*¹*)** |
|---|---|
| Black bull Liquid laundry detergent | 90 |
| Microcapsules *²* | 0.5-3.0 |
| DI-Water | Qs |
| Total | 100 |

| | |
|---|---|
| *¹* wt% relative to the total weight of the composition. *²* Encapsulated amount depends on the fragrance loading to 0.2% NOE. | |

Composition D is an example of powder detergent composition according to the present invention. It is prepared by admixture of the components described in Table 13, in the proportions indicated.

**Table 13**

| **Ingredient** | **Composition D (wt%*¹*)** |
|---|---|
| Linear alkylbenzenesulfonate | 22 |
| C₁₂₋₁₄ dimethylhydroxyethyl ammonium chloride | 0.2 |
| AE3S | 1 |
| Zeolite A | 1 |
| 1.6R silicate (SiO₂:Na₂O at ratio 1.6:1) | 5 |
| Sodium carbonate | 20 |
| Polyacrylate MW 4500 | 0.6 |
| Carboxymethyl cellulose | 0.3 |
| STAINZYME^{®} (20mg active/g) | 0.2 |
| Protease (SAVINASE^{®}, 32.89 mg active/g) | 0.1 |
| Lipase-LIPEX^{®} (18 mg active/g) | 0.07 |
| Fluorescent brightener | 0.06 |
| DTPA | 0.8 |
| MgSO₄ | 1 |
| Sodium percarbonate | 5.2 |
| TAED | 1.2 |
| Neat Fragrance | 0.5 |
| Microcapsules *²* | 0.5-3.0 |
| Total | 100 |

| | |
|---|---|
| *¹* wt% relative to the total weight of the composition. *²* Encapsulated amount depends on the fragrance loading to 0.2% NOE. | |

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure. Although the invention may be described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. Biodegradable core-shell microcapsules, wherein:
(i) the shell comprises chitosan cross-linked with a polyfunctional isocyanate having at least two isocyanate functional groups and a tannic acid, wherein the tannic acid is hydrolyzed tannic acid, unhydrolyzed tannic acid, or a combination thereof, preferably hydrolyzed tannic acid; and
(ii) the core comprises an active material;
wherein the microcapsules shell has a biodegradation rate of at least 20%, 30%, 40%, 50%, 60%, 65%, 70%, 75%, 80%, 85%, 90%, 95% or 98%, within 60 days according to OECD301F or OECD310.

2. The biodegradable core-shell microcapsules of claim 1, wherein:
(a) the shell further comprises carrageenan, gum Arabic (gum acacia), or a combination thereof; and/or
(b) the polyfunctional isocyanate is a biuret, isocyanurate, allophanate, uretdione, oligomeric hexamethylene diisocyanate, or a combination thereof.

3. The biodegradable core-shell microcapsules of claim 1 or 2, wherein:
(a) obtained slurry of the biodegradable core-shell microcapsules has a level of self-condensed polyfunctional isocyanate that is below 10%, below 5%, below 3%, below 1% or below 0.5%, relative to total weight of the polyfunctional isocyanate used to form wall of the microcapsules; or
(b) materials used to form wall of the microcapsules do not form a blend of biodegradable materials and non-biodegradable materials; or
(c) materials used to form wall of the microcapsules forms a blend of biodegradable materials and non-biodegradable materials, wherein levels of the non-biodegradable materials are below 10%, below 5%, below 3%, below 1% or below 0.5%, relative to weight of the microcapsules; or
(d) materials used to form the microcapsules forms a blend of biodegradable materials and non-biodegradable materials, wherein the biodegradation rate of all components of the blend is at least 20%, 30%, 40%, 50%, 60%, 65%, 70%, 75%, 80%, 85%, 90%, 95% or 98%, within 60 days according to OECD301F or OECD310.

4. The biodegradable core-shell microcapsules of any one of the preceding claims, wherein the shell is further cross-linked with an amino acid, preferably the amino acid is selected from the group consisting of lysine, arginine, ornithine, cysteine, serine, threonine, asparagine, glutamine, and a combination thereof, more preferably the amino acid is lysine.

5. The biodegradable core-shell microcapsules of any one of the preceding claims, wherein the chitosan is derived from shellfish chitosan or fungal chitosan, preferably fungal chitosan, and preferably the microcapsules have a bio-renewable carbon (BRC) content of at least at least 50%, at least 55%, at least 60%, at least 65%, at least 70%, at least 75%, at least 80%, at least 85%, at least 90% or at least 95%.

6. The biodegradable core-shell microcapsules of any one of the preceding claims, wherein the active material comprises a fragrance, flavor, agricultural active, pesticide, pharmaceutical active, nutraceutical active, animal nutrition active, food active, microbio active, malodor counteractant, and/or cosmetic active, preferably a fragrance, more preferably a fragrance comprising at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2, and optionally at least one additional fragrance ingredient.

7. A process for making biodegradable core-shell microcapsules comprising:
(a) emulsifying (i) an aqueous solution comprising chitosan with (ii) an oil phase comprising an active material to form an emulsion; and
(b) cross-linking the chitosan by adding to the emulsion an isocyanate and a tannic acid thereby forming biodegradable core-shell microcapsules;
preferably the chitosan is fungal chitosan, the isocyanate is a polyfunctional isocyanate having at least two isocyanate functional groups and the tannic acid is hydrolyzed tannic acid, unhydrolyzed tannic acid, or a combination thereof.

8. The process of claim 7, wherein the aqueous solution further comprises carrageenan, gum arabic (gum acacia), or a combination thereof.

9. The process of claim 7 or 8, wherein the polyfunctional isocyanate is a biuret, isocyanurate, allophanate, uretdione, oligomeric hexamethylene diisocyanate, or a combination thereof, preferably the isocyanate is dispersed in a solvent, more preferably ethyl acetate, 3-methoxybutyl acetate, diethylene glycol monoethyl ether acetate (DGMEA), Propylene glycol diacetate, diethyl malonate, triacetin, triethyl citrate, ethyl acetoacetate, benzyl acetone, butyl carbitol acetate or any combination thereof.

10. The process of any one of claims 7 to 9, wherein the shell is further cross-linked with an amino acid, preferably the amino acid is selected from the group consisting of lysine, arginine, ornithine, cysteine, serine, threonine, asparagine, glutamine, and a combination thereof, preferably lysine.

11. The process of any one of claims 7 to 10, wherein the active material comprises a fragrance, flavor, agricultural active, pesticide, pharmaceutical active, nutraceutical active, animal nutrition active, food active, microbio active, malodor counteractant, and/or cosmetic active, preferably a fragrance, more preferably a fragrance comprising at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2, and optionally at least one additional fragrance ingredient.

12. Biodegradable core-shell microcapsules obtainable by the process of any one of claims 7 to 11.

13. A consumer product comprising the biodegradable core-shell microcapsules of any one of claims 1 to 6, or claim 12, preferably the consumer product is a fabric softener, fabric conditioner, detergent, scent booster, fabric refresher spray, body wash, body soap, shampoo, hair conditioner, body spray, hair refresher spray, hair dye, hair moisturizer, skin moisturizer, hair treatment, skin treatment, antiperspirant, deodorant, insect repellant, candle, surface cleaner, bathroom cleaner, bleach, cat litter, or refresher spray.

14. Biodegradable core-shell microcapsules, wherein the shell comprises a biopolymer cross-linked with an isocyanate and a hydrolyzed tannic acid and the core comprises an active material, wherein the microcapsules shell has a biodegradation rate of at least 60% within 60 days according to OECD301F or OECD310, and the active material comprises a fragrance, flavor, agricultural active, pesticide, pharmaceutical active, nutraceutical active, animal nutrition active, food active, microbio active, malodor counteractant, and/or cosmetic active, preferably a fragrance, more preferably a fragrance comprising at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2, and optionally at least one additional fragrance ingredient.

15. The biodegradable core-shell microcapsules of claim 14, wherein the biopolymer comprises:
(i) a chitosan, pectin, chitosan oligosaccharide, carrageenan, modified guar, modified glucan, gum arabic (gum acacia), modified gum arabic (modified gum acacia), alginate, modified starch, modified cellulose, or a combination thereof, preferably the biopolymer is fungal chitosan; or
(ii) a protein, gelatin, collagen, glucoamylase, hydrolyzed protein, fermented protein, hydrophobin, enzyme, partially neutralized citric acid ester; or
(iii) a combination of (i) and (ii).
